# EUROPÄISCHE PATENTSCHRIFT

(11) **EP 3 538 176 B1**
(45) Veröffentlichungstag und Bekanntmachung des Hinweises auf die Patenterteilung: **29.03.2023**
(21) Anmeldenummer: 17801594.7
(22) Anmeldetag: 08.11.2017
(51) Int. Cl.: A61M 1/16

(54) **DIALYSEGERÄT**
DIALYSIS MACHINE
APPAREIL DE DIALYSE

(30) Priorität: 08.11.2016 DE 102016013316
(43) Veröffentlichungstag der Anmeldung: 18.09.2019
(73) Patentinhaber: Fresenius Medical Care Deutschland GmbH, 61352 Bad Homburg (DE)
(72) Erfinder: IRRGANG, Tobias, 97633 Aubstadt (DE)
(74) Vertreter: Herrmann, Uwe
(86) Internationale Anmeldenummer: PCT/EP2017/001303
(87) Internationale Veröffentlichungsnummer: WO 2018/086739

(56) Entgegenhaltungen:
- EP-A1- 0 714 668
- EP-A2- 0 367 252
- WO-A1-2012/052151
- WO-A2-2017/054923
- DE-A1-102015 012 604
- US-A- 4 770 769

## Beschreibung

Die Erfindung betrifft ein Dialysegerät mit einem extrakorporalen Blutsystem, einem Dialysierflüssigkeitssystem, einem darin angeordneten Dialysator und einer Steuereinheit, wobei das Dialysegerät des Weiteren eine Bilanzkammer mit zwei Bilanzkammerhälften zum bilanzierten Zu- und Abführen von Dialysierflüssigkeit zu und von dem Dialysator umfasst, wobei eine Leitung vorgesehen ist, durch die Dialysierflüssigkeit zu der Bilanzkammer geführt wird und wobei eine Mischkammer vorgesehen ist, in der sich die frische Dialysierflüssigkeit oder ein Bestandteil von dieser befindet und die unmittelbar oder mittelbar mit einer durch ein Ventil verschließbaren Entlüftungsleitung in Verbindung steht.

Offenbart ist des Weiteren ein Verfahren zum Betreiben eines derartigen Dialysegerätes.

Die Druckschrift WO2012/052151A1 offenbart ein Dialysegerät, mittels dessen aus mit Luft übersättigtem Wasser austretende Gasblasen entfernt werden können. Aus der Druckschrift EP0714668A1 ist eine Aufbereitungsanlage für Salzlösung für die Dialyse bekannt. Weiterhin ist aus der Druckschrift WO2017/054923A2 ein weiteres Dialysegerät bekannt.

Dialysegeräte gemäß dem Oberbegriff des Anspruchs 1 sind aus dem Stand der Technik bekannt.

Bekannte Dialysegeräte werden im Rahmen einer Dialysebehandlung dazu verwendet, Harnstoff und andere Stoffe aus dem Blut eines Patienten mit fehlender oder verminderter Nierenaktivität zu entfernen. Das zentrale Element eines Dialysegeräts ist der Dialysator, wobei es sich um eine Filtereinheit mit einer Blutkammer und einer Dialysierflüssigkeitskammer handelt, die durch eine semipermeable Membran getrennt sind. Die aus dem Blut zu entfernenden Stoffe treten im Dialysator durch die semipermeable Membran von der Blutkammer in die Dialysierflüssigkeitskammer über.

Zum Zwecke der Bilanzierung der dem Dialysator zugeführten und der von diesem abgeführten Menge an Dialysierflüssigkeit wird eine sogenannte Bilanzkammer eingesetzt. Diese weist zwei durch eine bewegliche Wand voneinander getrennte Bilanzkammerhälften auf, die jeweils über einen Zulauf und über einen Ablauf verfügen, die über je ein Ventil geöffnet und geschlossen werden können. Während eine der Bilanzkammerhälften durch frische Dialysierflüssigkeit gefüllt wird, bewegt sich die Wand von der sich füllenden Bilanzkammerhälfte weg und verdrängt auf diese Weise die in der anderen Bilanzkammerhälfte befindliche gebrauchte Dialysierflüssigkeit. Da sich beide Bilanzkammerhälften in derselben Bilanzkammer mit festen Wandungen befinden, entspricht das zugeführte Volumen an frischer Dialysierflüssigkeit dem abgeführten Volumen an verbrauchter Dialysierflüssigkeit.

Aus dem Stand der Technik ist es des Weiteren bekannt, die Dialysierflüssigkeit am Gerät selbst durch den Einsatz von Konzentratbehältern herzustellen, deren Konzentrate mit RO-Wasser gemischt werden. Dabei werden üblicherweise zwei Konzentratbehälter verwendet, von denen einer ein basisches und einer ein saures Konzentrat enthält. Die Konzentrate werden mittels Konzentratpumpen in eine Mischkammer gefördert, in der sie mit RO-Wasser gemischt werden. Diese Mischung wird sodann in zu der Bilanzkammer gefördert und von dort aus zum Dialysator geleitet.

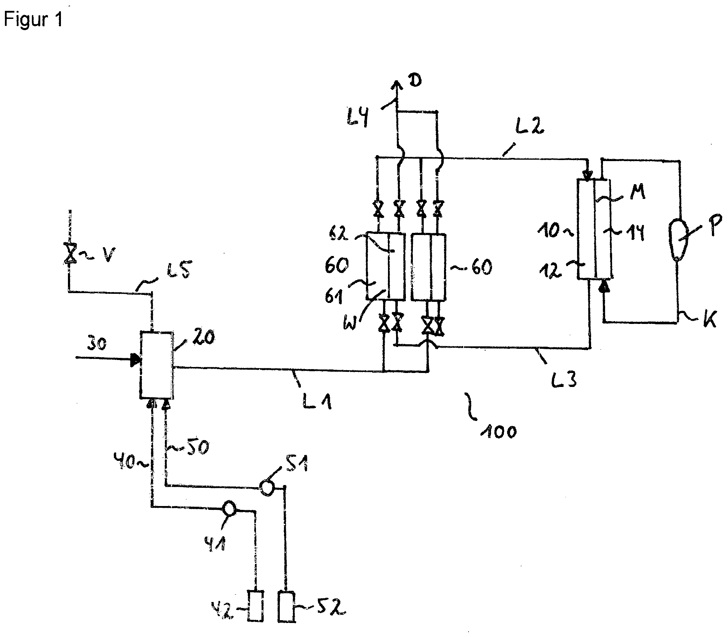

Ein derartiges aus dem Stand der Technik bekanntes Dialysegerät ist in Figur 1 stark vereinfacht dargestellt. Der Patient P steht über den extrakorporalen Kreislauf K mit dem Dialysator 10 in Verbindung, der ein blutseitiges 14 und ein dialysatseitiges Kompartiment 12 aufweist, die durch eine semipermeable Membran M voneinander getrennt sind. Das Blut des Patienten wird im Gegenstrom zu der Dialysierflüssigkeit durch den Dialysator 10 geleitet, wie dies durch Pfeile angedeutet ist, die die Strömungsrichtung wiedergeben.

Das hydraulische System des Dialysegerätes, d.h. das Dialysierflüssigkeitssystem ist allgemein mit dem Bezugszeichen 100 gekennzeichnet.

Die Dialysierflüssigkeit wird in der Mischkammer 20 aus der durch die Leitung 30 einströmenden Flüssigkeit, vorzugsweise RO-Wasser (RO = Reversosmose) sowie aus den beiden Konzentraten hergestellt, die durch die Leitungen 40, 50 in die Mischkammer 20 einströmen. Die Förderung der Konzentrate erfolgt mittels der Pumpen 41, 51, die das Konzentrat aus den Konzentratbehältern 42, 52 fördern.

Die an der Mischkammer 20 angeordnete Leitung L5 dient zur Entlüftung der Dialysierflüssigkeit und ist durch das Ventil V verschließbar.

Die Bilanzkammer 60 weist die oben genannten Bilanzkammerhälften 61 und 62 auf, die durch die bewegliche, nicht für Dialysierflüssigkeit permeable Wand W voneinander getrennt sind.

Um einen kontinuierlichen Fluss zu gewährleisten, sind zwei Bilanzkammern 60 vorgesehen, wie dies aus Figur 1 hervorgeht. Diese können identisch aufgebaut sein.

Die Bilanzkammern sind insofern vereinfacht dargestellt, als dass deren Innenwandungen vorzugsweise so ausgeformt sind, dass sich die Wand W in ihrem Extrempositionen vollständig an diese anlegen kann.

Wie dies weiter aus Figur 1 hervorgeht, weist jede Bilanzkammerhälfte 61, 62 einer Bilanzkammer 60 je ein Ventil auf der Ein- und auf der Auslassseite auf, um den Zu- und Ablauf in die und aus den Bilanzkammerhälften zu steuern.

Die beiden links dargestellten Bilanzkammerhälften 61 der Bilanzkammern 60 dienen zur Aufnahme frischer Dialysierflüssigkeit aus der Leitung L1. Aus den Bilanzkammerhälften 61 gelangt die Dialysierflüssigkeit über die Leitung L2 in das dialysatseitige Kompartiment 12 des Dialysators 10.

Die beiden rechts dargestellten Bilanzkammerhälften 62 der Bilanzkammern 60 dienen zur Aufnahme verbrauchter Dialysierflüssigkeit, die über die Leitung L3 von dem Dialysator 10 zu den Bilanzkammerhälften 62 gelangt und nach der Bilanzkammer über die Leitung L4 zu einem Ablauf (Drain D) geführt wird.

In den Fällen, in denen sich in den Konzentratleitungen 40, 50 keine Luftabscheider befinden, kann der Fall eintreten, dass Luft in die Bilanzkammer gelangt, was zu einem Bilanzierfehler führt, da die Bilanzkammerhälfte der Bilanzkammer dann nicht vollständig mit frischer Dialysierflüssigkeit befüllt wird und somit weniger frische Dialysierflüssigkeit zum Dialysator gefördert wird, als angenommen.

Das Eintreten von Luft in die Bilanzkammer bzw. in die Konzentratleitungen kann beispielsweise dann erfolgen, wenn einer oder beide der Konzentratbehälter leer sind. In diesen Fällen wird Luft über die Konzentratleitungen in die Mischkammer und von dort aus in die Bilanzkammer gefördert bzw. mitgerissen.

Dieses Mitreißen der Luft erfolgt durch das Öffnen des Entlüftungsventils V zur Totzeit der Bilanzkammer. Unter der Totzeit der Bilanzkammer ist die Zeitspanne zu verstehen, in der sich die Wand W in einer Extremlage (Auslenkung ganz nach rechts oder links) befindet und alle Ventile der Bilanzkammer geschlossen sind. Dieser Zustand kann beispielsweise 100 ms andauern.

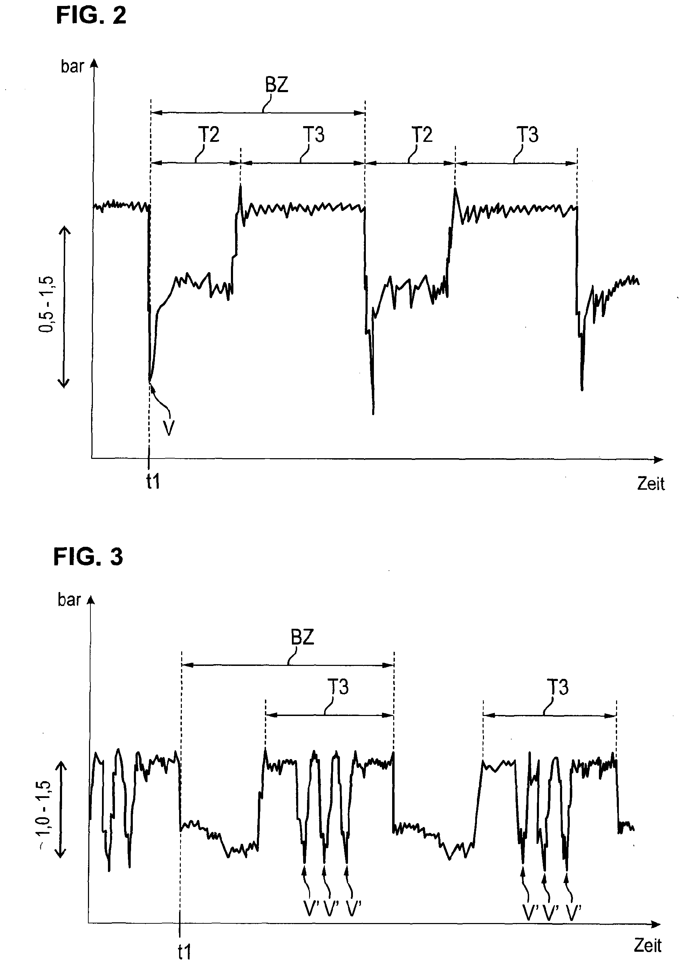

Figur 2 zeigt den zeitlichen Druckverlauf während eines Bilanzkammerzyklus, wobei der Zeitpunkt t1 die genannte Totzeit kennzeichnet. Wie aus Figur 2 durch einen Pfeil V gekennzeichnet, wird zu diesem Zeitpunkt t1 das Ventil V kurzzeitig geöffnet. Anschließend wird das Ventil V geschlossen und das Zulaufventil der Bilanzkammer geöffnet, so dass der Druck in der sich füllenden Bilanzkammerhälfte 61 ansteigt. Diese Füllphase ist in Figur 2 mit T2 gekennzeichnet. In dem sich daran anschließenden Zustand T3 ist die Bilanzkammerhälfte vollständig gefüllt, was auch als Hochdruckphase der Bilanzkammer bezeichnet wird.

Das Bezugszeichen BZ kennzeichnet den gesamten Bilanzkammerhälftenzyklus, der alle diese Phasen bzw. Zeitpunkte umfasst.

Die Luft steigt bei offenem Ventil V in den Leitungen 40, 50 nach oben und wird dann durch den Füllfluss, mittels dessen die Bilanzkammer 61 gefüllt wird, in die Bilanzkammer bzw. Bilanzkammerhälfte 61 mitgerissen. Dies führt zu einer Fehlbilanz.

Wie oben ausgeführt, kann das Öffnen des Ventils V zu einer Ansammlung von Luft in der Bilanzkammer führen, was unerwünscht ist, da dann keine korrekten Flüssigkeitsvolumina gefördert werden.

Der vorliegenden Erfindung liegt daher die Aufgabe zugrunde, ein Dialysegerät gemäß dem Oberbegriff des Anspruchs 1 dahingehend weiterzubilden, dass ein Lufteintrag in die Bilanzkammer verhindert oder gegenüber bekannten Geräten zumindest deutlich verringert wird.

Diese Aufgabe wird durch ein Dialysegerät mit den Merkmalen des Anspruchs 1 gelöst.

Danach ist vorgesehen, dass das Dialysegerät über eine Steuereinheit verfügt, die ausgebildet ist, dass die Steuereinheit in dem Betriebszustand der Spezialluftabscheidung des Dialysegerätes das Ventil ein- oder vorzugsweise mehrmals öffnet und schließt, wenn sich die mit der genannten Leitung (Leitung L1) in Fluidverbindung stehende Bilanzkammerhälfte der Bilanzkammer in ihrer Hochdruckphase befindet.

Während dieser im Rahmen der vorliegenden Erfindung auch als "Spezialluftabscheidung" bezeichneten Vorgehensweise ist die betreffende Bilanzkammerhälfte gefüllt und das Ventil wird vorzugsweise mehrfach geöffnet und geschlossen. Da aufgrund der vollen Bilanzkammerhälfte kein Fluss mehr in Richtung zu der Bilanzkammer stattfindet, wird bei diesem Vorgang keine Luft mitgerissen.

Durch das vorzugsweise mehrfache, beispielsweise dreifache Öffnen und Schließen des Ventils wir die Luft mechanisch ausgetrieben, so dass weniger Restluft in der Mischkammer verbleibt, die bei der nächsten Füllung der Bilanzkammer mitgerissen werden könnte.

Insgesamt wird auf diese Weise die Wahrscheinlichkeit für das Einbringen von Luft in die Bilanzkammer wesentlich verringert.

Folgt auf diese Spezialluftabscheidung wieder eine normale Luftabscheidung, wie sie in Figur 2 gezeigt ist, wird beim Öffnen des Ventils V keine oder sehr viel weniger Luft in die Bilanzkammer mitgerissen, als dies beim Stand der Technik der Fall ist, weil sich in der Bilanzkammer keine oder nur noch sehr wenig Luft befindet. Vorzugsweise steht die Mischkammer mit einer oder mehreren Konzentratleitungen in Verbindung, durch die Konzentrate aus Konzentratbehältern in die Mischkammer gelangen. Bei diesen Konzentraten kann es sich beispielsweise um ein saures und um ein basisches Konzentrat handeln.

Üblicherweise steht die Mischkammer mit einem Frischwasseranschluss in Verbindung, insbesondere mit einem RO-Wasseranschluss, so dass das oder die Konzentrate auf das gewünschte Niveau verdünnt werden können.

Das Dialysegerät weist Erkennungsmittel auf, die ausgebildet sind, das Fehlen oder einen Leerzustand eines Konzentratbehälters zu erfassen und die mit der Steuereinheit in Verbindung stehen, so dass diese den Betriebszustand der Spezialluftabscheidung einleitet, wenn die genannte Erfassung durch die Erkennungsmittel erfolgt ist.

Erfassen die Erkennungsmittel beispielsweise einen Leerzustand eines Konzentratbehälters, worunter nicht nur zu verstehen ist, dass der Behälter ganz leer ist, sondern auch der Zustand, dass der Füllstand unter einem bestimmten Niveau liegt, wird davon ausgegangen, dass vergleichsweise viel Luft in der Mischkammer oder den mit diesen in Verbindung stehenden Leitungen vorhanden ist, so dass der Betriebsmodus der Spezialluftabscheidung eingeleitet wird, bei dem das Ventil wenigstens einmal, vorzugsweise mehrfach während der Hochdruckphase der Bilanzkammer bzw. Bilanzkammerhälfte geöffnet und geschlossen wird.

Die Steuereinheit kann derart ausgebildet sein, dass diese einen normalen Luftabscheidezyklus durchführt, wenn seitens der Erkennungsmittel weder das Fehlen noch ein Leerzustand eines Konzentratbehälters erfasst wird, wobei der normale Luftabscheidezyklus in einem Öffnen des Ventils in der Totzeit der Bilanzkammer besteht, wie dies in Figur 2 ersichtlich ist. In diesem Zustand erfolgen vorzugsweise keine Öffnungen und Schließungen des Ventils in einem auf die Totzeit folgenden Zeitpunkt, insbesondere nicht in der Hochdruckphase.

Üblicherweise ist der normale Luftabscheidemodus der Normalzustand, während der Spezialluftabscheidemodus nur dann gewählt wird, wenn das Fehlen oder der Leerzustand eines Konzentratbehälters erkannt wird.

Die vorliegende Erfindung kommt vorzugsweise bei Dialysegeräten zum Einsatz, die über keine Luftabscheidevorrichtung verfügen.

Offenbart ist des Weiteren ein Verfahren zum Betreiben eines Dialysegerätes nach einem der vorhergehenden Ansprüche, wobei in dem Betriebszustand der Spezialluftabscheidung des Dialysegerätes das Ventil einoder mehrmals geöffnet und geschlossen wird, wenn sich die mit der Leitung in Fluidverbindung stehende Bilanzkammerhälfte der Bilanzkammer in ihrer Hochdruckphase befindet.

Wie oben ausgeführt, wird der Betriebszustand der Spezialluftabscheidung vorzugsweise eingestellt, wenn ein Konzentratbehälter fehlt oder leer ist oder andere Gründe vorliegen, bei denen damit zu rechnen ist, dass vergleichsweise viel Luft in der Mischkammer anfällt.

Des Weiteren ist vorzugsweise vorgesehen, dass der Betriebszustand der Spezialluftabscheidung nicht gewählt wird, wenn kein Konzentratbehälter fehlt oder leer ist, sondern dass in diesem Fall eine normale Luftabscheidung vorgenommen wird, die ein Öffnen des Ventils nur in der Totzeit der Bilzanzkammer vorsieht.

Weitere Einzelheiten und Vorteile der Erfindung ergeben sich aus den nachfolgend anhand der Figuren dargestellten Ausführungsbeispielen. In den Figuren zeigen:
- Figur 1:: eine vereinfachte schematische Darstellung des Hydraulik- und Blutkreislaufes eines Dialysegerätes;
- Figur 2:: den Verlauf des Druckes über die Zeit bei einer normalen Luftabscheidung und
- Figur 3:: den Verlauf des Druckes über die Zeit bei einer Spezialluftabscheidung.

Figur 1 zeigt einen hydraulischen Kreislauf sowie auch den extrakorporalen Blutkreislauf eines aus dem Stand der Technik bekannten Dialysegerätes. Dieser Aufbau kann identisch oder auch in abgewandelter Form auch bei einem Dialysegerät gemäß der Erfindung vorliegen, so dass die obigen Ausführungen auch für die vorliegende Erfindung gelten.

Im Rahmen des normalen Betriebs wird die normale Luftabscheidung durchgeführt, wie sie oben beschrieben und in Figur 2 dargestellt ist. Die Luftabscheidung erfolgt durch Öffnen des Ventils V und zwar zum Totzeitpunkt der Bilanzkammer bzw. der mit frischer Dialysierflüssigkeit gefüllten Bilanzkammerhälfte, anschließend während des Bilanzkammerzyklus BZ nicht mehr.

Wird allerdings durch geeignete Erkennungsmittel, wie z.B. Sensoren erkannt, dass übermäßiger Lufteintrag stattfindet, was z.B. durch das Fehlen eines Konzentratbehälters 42, 52 oder dessen Leerzustand bedingt sein kann oder durch einen übermäßig großen Luftgehalt in der Mischkammer oder einer der Leitungen, wird dies an eine nicht dargestellte Steuereinheit gemeldet, die daraufhin die Spezialluftabscheidung einleitet.

Diese zeichnet sich dadurch aus, dass in der Hochdruckphase der mit frischer Dialysierflüssigkeit gefüllten Bilanzkammerhälfte das Ventil V gemäß Figur 1 einmal oder vorzugsweise mehrfach, z.B. dreimal geöffnet wird.

In der Hochdruckphase der Bilanzkammerhälfte ist diese vollständig mit Flüssigkeit gefüllt. Dies hat zur Folge, dass das Öffnen des Ventils V nicht zu einer Strömung von mit Luft angereicherter Flüssigkeit in die Bilanzkammer führt. Vielmehr wird durch das Öffnen und Schließen des Ventils Luft aus der Mischkammer 20 entfernt, so dass bei dem Zurückschalten in den normalen Betriebsmodus bzw. normalen Luftabscheidemodus keine oder nur wenig Luft in die Bilanzkammer gelangt. Dadurch lässt sich die Wahrscheinlichkeit für das Auftreten von Fehlbilanzierungen vermeiden oder zumindest verringern.

Figur 3 zeigt den Verlauf des Druckes über die Zeit in einer Anordnung gemäß Figur 2, wobei gleiche Zeitpunkte und Zeitspannen mit denselben Bezugszeichen versehen sind, und verdeutlicht, dass in der Hochdruckphase T2 das Ventil V dreimal geöffnet und geschlossen wird, was durch das Bezugszeichen V' gekennzeichnet ist und sich durch entsprechende Druckschwankungen bemerkbar macht. Ein Öffnen und Schließen des Ventils V zum Totzeitpunkt t1 findet vorzugsweise nicht statt.

Die in Figur 2 und 3 angegebenen Druckwerte sind exemplarischer Natur und nicht beschränkend.

Abschließend wird darauf hingewiesen, dass die Verwendung des Begriffs "ein" oder "eine" zwar auch aber nicht zwingend bedeutet, dass genau eines der fraglichen Elemente vorgesehen ist, sondern auch die Mehrzahl von Elementen einschließt. Ebenso ist darauf hinzuweisen, dass die Verwendung der Mehrzahl grundsätzlich auch eines der fraglichen Elemente mit einschließt.

## Patentansprüche

1. Dialysegerät mit einem extrakorporalen Blutsystem, einem Dialysierflüssigkeitssystem mit einem Dialysator, mit einer Steuereinheit, wobei das Dialysegerät des Weiteren eine Bilanzkammer mit zwei Bilanzkammerhälften zum bilanzierten Zu- und Abführen von Dialysierflüssigkeit zu und von dem Dialysator umfasst, wobei eine Leitung vorgesehen ist, durch die Dialysierflüssigkeit zu der Bilanzkammer geführt wird und wobei stromaufwärts der Bilanzkammer eine Mischkammer (20) vorgesehen ist, in der sich die frische Dialysierflüssigkeit oder ein Bestandteil von dieser befindet und die unmittelbar oder mittelbar mit einer durch ein Ventil (V) verschließbaren Entlüftungsleitung in Verbindung steht,
**dadurch gekennzeichnet,**
**dass** das Dialysegerät Erkennungsmittel aufweist, die dazu ausgebildet sind, das Fehlen oder einen Leerzustand eines Konzentratbehälters (42,52) zu erfassen, und die mit der Steuereinheit in Verbindung stehen, wobei die Steuereinheit ausgebildet ist, wenn die genannte Erfassung durch die Erkennungsmittel erfolgt ist, das Ventil (V) ein- oder mehrmals zu öffnen und zu schließen, wenn sich die mit der genannten Leitung in Fluidverbindung stehende Bilanzkammerhälfte der Bilanzkammer in ihrer Hochdruckphase befindet.

2. Dialysegerät nach Anspruch 1, **dadurch gekennzeichnet, dass** die Mischkammer (20) mit Konzentratleitungen in Verbindung steht, durch die Konzentrate aus Konzentratbehältern (42,52) in die Mischkammer (20) gelangen.

3. Dialysegerät nach einem der vorhergehenden Ansprüche, **dadurch gekennzeichnet, dass** die Mischkammer (20) mit einem Frischwasseranschluss, insbesondere mit einem RO-Wasseranschluss in Verbindung steht.

4. Dialysegerät nach Anspruch 1, **dadurch gekennzeichnet, dass** die Erkennungsmittel ausgebildet sind, zu erkennen, wenn ein Konzentratbehälter (42,52) nicht angeschlossen oder leer ist oder einen bestimmten Füllstand unterschritten hat.

5. Dialysegerät nach Anspruch 4 , **dadurch gekennzeichnet, dass** die Steuereinheit derart ausgebildet ist, dass diese einen normalen Luftabscheidezyklus durchführt, wenn seitens der Erkennungsmittel weder das Fehlen noch ein Leerzustand eines Konzentratbehälters (42,52) erfasst wird, wobei der normale Luftabscheidezyklus in einem Öffnen des Ventils (V) in der Totzeit der Bilanzkammer besteht.

## Claims

1. Dialysis machine having an extracorporeal blood system; a dialysis fluid system having a dialyzer; having a control unit, wherein the dialysis machine furthermore comprises a balancing chamber having two balancing chamber halves for a balanced infeed and draining of dialysis fluid to and from the dialyzer, wherein a line is provided through which the dialysis fluid is conducted to the balancing chamber, and wherein a mixing chamber (20) is provided upstream of the balancing chamber in which the fresh dialysis fluid or a component thereof is located and which is directly or indirectly connected to a de-airing line closable by a valve (V),
**characterized in that**
the dialysis machine comprises recognition means which are configured to detect the lack or an empty state of a concentrate container (42,52), and which are connected to the control unit, wherein the control unit is configured, when the named detection by the recognition means occured, to open and close the valve (V) once, or a number of times, when the balancing chamber half of the balancing chamber in fluid communication with the named line is in its high-pressure phase.

2. Dialysis machine in accordance with claim 1, **characterized in that** the mixing chamber (20) is connected to concentrate lines through which concentrates move out of concentrate containers (42,52) into the mixing chamber (20).

3. Dialysis machine in accordance with one of the preceding claims, **characterized in that** the mixing chamber (20) is connected to a fresh water connection, in particular to an RO water connection.

4. Dialysis machine in accordance with claim 1, **characterized in that** the recognition means are configured to recognize when a concentrate container (42,52) is not connected or is empty or has fallen below a specific filling level.

5. Dialysis machine in accordance with claim 4, **characterized in that** the control unit is configured such that it carries out a normal air separation cycle when neither the lack nor an empty state of a concentrate container (42,52) is detected on the part of the recognition means, wherein the normal air separation cycle consists in an opening of the valve (V) in the dead time of the balancing chamber.

## Revendications

1. Appareil de dialyse avec un système sanguin extracorporel, un système de liquide de dialyse avec un dialyseur, avec une unité de commande, l'appareil de dialyse comprenant en outre une chambre d'équilibre avec deux moitiés de chambre d'équilibre pour l'amenée et l'évacuation équilibrées de liquide de dialyse vers et depuis le dialyseur, une conduite étant prévue, par laquelle le liquide de dialyse est amené à la chambre d'équilibre et une chambre de mélange (20) étant prévue en amont de la chambre d'équilibre, dans laquelle se trouve le liquide de dialyse frais ou un composant de celui-ci et qui est en liaison directe ou indirecte avec une conduite d'aération pouvant être fermée par une soupape (V),
**caractérisé en ce que**
l'appareil de dialyse présente des moyens de détection qui sont configurés pour détecter l'absence ou un état vide d'un réservoir de concentré (42, 52) et qui sont en communication avec l'unité de commande, l'unité de commande étant configurée, lorsque ladite détection est effectuée par les moyens de détection, pour ouvrir et fermer la soupape (V) une ou plusieurs fois lorsque la moitié de chambre d'équilibrage de la chambre d'équilibrage en communication fluidique avec ladite conduite est dans sa phase haute pression.

2. Appareil de dialyse selon la revendication 1, **caractérisé en ce que** la chambre de mélange (20) est en liaison avec des conduites de concentrés par lesquelles des concentrés provenant de réservoirs de concentrés (42, 52) parviennent dans la chambre de mélange (20).

3. Appareil de dialyse selon l'une quelconque des revendications précédentes, **caractérisé en ce que** la chambre de mélange (20) est en liaison avec un raccordement d'eau fraîche, notamment avec un raccordement d'eau OI.

4. Appareil de dialyse selon la revendication 1, **caractérisé en ce que** les moyens de détection sont configurés pour détecter lorsqu'un réservoir de concentré (42, 52) n'est pas raccordé ou est vide ou est passé en dessous d'un certain niveau de remplissage.

5. Appareil de dialyse selon la revendication 4, **caractérisé en ce que** l'unité de commande est configurée de telle sorte que celle-ci réalise un cycle normal de séparation de l'air lorsque ni l'absence ni un état vide d'un réservoir de concentré (42, 52) n'est détecté par les moyens de détection, le cycle normal de séparation de l'air consistant en une ouverture de la soupape (V) pendant le temps mort de la chambre d'équilibre.
